# EUROPÄISCHE PATENTSCHRIFT

(11) **EP 2 539 006 B1**
(45) Veröffentlichungstag und Bekanntmachung des Hinweises auf die Patenterteilung: **14.09.2016**
(21) Anmeldenummer: 11706221.6
(22) Anmeldetag: 25.02.2011
(51) Int. Cl.: A61M 11/00, A61M 16/12, H04R 1/10, A61M 16/00, A61M 16/08, A61M 16/06, A61M 16/16

(54) **VORRICHTUNG ZUM BEFEUCHTEN DER ATEMLUFT EINES BENUTZERS**
DEVICE FOR HUMIDIFYING THE RESPIRATORY AIR OF A USER
DISPOSITIF POUR L'HUMIDIFICATION DE L'AIR DE RESPIRATION D'UN UTILISATEUR

(30) Priorität: 27.02.2010 DE 102010009666
(43) Veröffentlichungstag der Anmeldung: 02.01.2013
(73) Patentinhaber: Fraunhofer-Gesellschaft zur Förderung der angewandten Forschung e.V., 80686 München (DE)
(72) Erfinder: SCHWAB, Rudolf, 83551 Lenggries (DE)
(74) Vertreter: Friese Goeden Patentanwälte
(86) Internationale Anmeldenummer: PCT/EP2011/052785
(87) Internationale Veröffentlichungsnummer: WO 2011/104324

(56) Entgegenhaltungen:
- WO-A2-01/87394
- FR-A1- 2 752 165
- US-A- 6 065 473

## Beschreibung

Die Erfindung betrifft eine Vorrichtung zum Befeuchten der Atemluft eines Benutzers, enthaltend eine Kopfhalterung, welche am Kopf eines Benutzers angeordnet werden kann, und welche sich von einer Ohrregion eines Benutzers zur anderen Ohrregion des Benutzers erstreckt; einen Arm, aufweisend ein erstes Ende und ein zweites Ende, wobei das erste Ende an der Kopfhalterung befestigt ist, und sich das zweite Ende in Richtung des Nasen- und/oder Mundbereiches des Benutzers erstreckt; und ein Austrittsteil, welches mit dem zweiten Ende des Armes verbindbar ist, und welches zumindest eine erste Öffnung enthält, durch welche ein erstes Fluid zu dem Benutzer geleitet werden kann

Aus der US 2008/0053457 A1 ist eine gattungsgemäße Vorrichtung bekannt. Diese bekannte Vorrichtung ist dazu vorgesehen, einem Patienten mit respiratorischer Insuffizienz Sauerstoff zuzuführen. Hierzu weist die Vorrichtung ein Rohr auf, welches mittels eines Kopfbügels so positioniert wird, dass das Rohrende vor den Atemöffnungen des Patienten positioniert ist. Aus dem Rohrende strömt bei Betrieb der Vorrichtung Sauerstoff aus, so dass sich im Bereich der Atemöffnungen des Patienten ein erhöhter Sauerstoffgehalt der Atemluft ergibt. Die vorbekannte Vorrichtung wird jedoch ausschließlich im medizinischtherapeutischen Bereich eingesetzt.

Die US 6,065,473 zeigt ebenfalls eine gattungsgemäße Vorrichtung, welche einen Kopfbügel umfasst, an welchem ein Ausströmelement befestigt ist, mit welchem dem Benutzer Sauerstoff berührungslos zugeführt werden kann. Die Versorgung mit verschiedenen Gasen in individueller Dosierung ist mit diesem Gerät jedoch nicht möglich.

Weiterhin ist aus der Praxis bekannt, dass insbesondere in Flugzeugen während des Reisefluges die Luftfeuchtigkeit sehr stark abnehmen kann. In Abhängigkeit der Belegungsdichte mit Passagieren kann die Luftfeuchtigkeit in einem Flugzeug auf 5 % relative Feuchte oder darunter sinken. Zwar sind Verfahren und Vorrichtungen bekannt, welche mittels der Verdampfung oder Vernebelung von Wasser die relative Luftfeuchte in einem umschlossenen Raum erhöhen können. Diese Verfahren sind jedoch in Flugzeugen nicht einsetzbar. So wird eine erhebliche Wassermenge benötigt, um das gesamte Luftvolumen eines Passagierflugzeuges zu befeuchten. Diese Wassermenge erhöht das Abfluggewicht und damit den Treibstoffverbrauch. Weiterhin kann die Feuchte in die Kabinenisolierung eindringen, wodurch Korrosion entstehen und/oder die Luftqualität durch Schimmelbildung beeinträchtigt werden kann. Eine ähnliche Problematik kann auch in beheizten Innenräumen von Gebäuden oder Fahrzeugen auftreten.

Die Erfindung wird durch die beitiegenden Ansprüche definiert.

Der Erfindung liegt daher die Aufgabe zugrunde, das respiratorische Dehydrieren von Lebewesen in Innenräumen zu verhindern oder zumindest zu reduzieren.

Die Aufgabe wird erfindungsgemäß gelöst durch eine Vorrichtung gemäß Anspruch 1 und eine Verwendung gemäß Anspruch 9. Zweckmäßige Weiterbildungen der Erfindung finden sich in den jeweiligen Unteransprüchen.

Erfindungsgemäß wird vorgeschlagen, die Qualität der Atemluft eines Benutzers in einem kleinräumigen Bereich vor dessen Atemöffnungen, meist also vor Mund und Nase, zu modifizieren. Die erfindungsgemäße Vorrichtung erlaubt dabei, den kleinräumigen Bereich vor der Atemöffnung des Benutzers bei Bewegungen des Benutzers nachzuführen, so dass dem Benutzer stets optimierte Atemluft zur Verfügung steht.

Hierzu weist die vorgeschlagene Vorrichtung eine Kopfhalterung nach Art eines Kopfhörers auf, welche den Kopf des Benutzers umschließt. An der Kopfhalterung ist weiterhin zumindest ein Arm mit einem ersten Ende und einem zweiten Ende angeordnet, wobei das zweite Ende an der Kopfhalterung befestigt ist. Das erste Ende dient zur Aufnahme eines Austrittsteiles, aus welchem zumindest ein erstes Fluid in den Raumbereich vor der Atemöffnung des Benutzers geleitet werden kann. Das Fluid kann beispielsweise Sauerstoff enthalten, gasförmiges Wasser, flüssiges Wasser bzw. ein Aerosol oder zumindest einen Duftstoff, welcher ebenfalls gasförmig oder als Aerosol vorliegen kann. In einigen Ausführungsformen der Erfindung kann dem Raumbereich vor einer Atemöffnung eine Mischung aus mehreren dieser Komponenten zugeführt werden, welche aus zumindest einer Öffnung des Austrittsteiles austreten.

In einigen Ausführungsformen kann die Kopfhalterung weiterhin zumindest einen Schallwandler tragen, mit welchem zumindest ein Audiosignal wiedergegeben werden kann. Auf diese Weise kann die vorgeschlagene Vorrichtung einerseits zum Modifizieren der Atemluft des Benutzers dienen und gleichzeitig die Funktion eines Kopfhörers wahrnehmen. Solche Kopfhörer werden üblicherweise auf Langstreckenflügen ohnehin an die Passagiere ausgegeben, um Audiosignale eines im Flugzeug vorhandenen Entertainmentsystems wiederzugegeben bzw. hörbar zu machen. Den Passagieren wird somit in einfacher Weise Gelegenheit gegeben, einerseits ein zerstreuendes Unterhaltungsprogramm wahrzunehmen und andererseits befeuchtete Luft zu atmen, welche dem Dehydrieren entgegenwirkt.

Da erfindungsgemäß nur ein unmittelbar vor den Atemöffnungen der Benutzer befindlicher, kleiner Raumbereich mit zusätzlicher Feuchte beaufschlagt wird, ist der Wasserverbrauch der erfindungsgemäßen Vorrichtung wesentlich niedriger als der Wasserverbrauch, welcher entstehen würde, wenn das gesamte Luftvolumen im Inneren des Passagierflugzeuges befeuchtet würde. Da der Großteil des Luftvolumens nach wie vor nur eine geringe relative Feuchte aufweist, kann sich die lokal eingebrachte Feuchte nicht in der Isolierung oder den Polstern des Flugzeuges anreichern, so dass ein Beschlagen der Fenster, eine Korrosion oder eine Schimmelbildung zuverlässig vermieden wird.

In einigen Ausführungsformen der Erfindung kann neben einer Befeuchtung der Atemluft auch eine Anreicherung mit Sauerstoff erfolgen. Auf diese Weise wird trotz der erforderlichen Druckabsenkung in der Kabine der Sauerstoffpartialdruck in etwa konstant gehalten, so dass das Wohlbefinden der Fluggäste erhöht wird.

Um eine individuelle Positionierung des Austrittsteiles zu ermöglichen, kann der Arm schwenkbar an der Kopfhalterung befestigt sein und/oder eine durch den Benutzer veränderliche Länge und/oder eine durch den Benutzer veränderliche Form aufweisen. Auf diese Weise kann der Benutzer durch Schwenken, Biegen oder Längenverstellen das Austrittsteil wunschgemäß positionieren, so dass beispielsweise eine Nahrungsaufnahme nicht behindert wird.

In einigen Ausführungsformen der Erfindung kann das Austrittsteil zumindest eine erste Öffnung aufweisen, durch welche ein erstes Fluid zu dem Benutzer geleitet werden kann und zumindest eine zweite Öffnung, durch welche ein zweites Fluid zu dem Benutzer geleitet werden kann. In diesem Fall kann die sich im Gasraum vor der Atemöffnung des Benutzers bildende Gaszusammensetzung individuell eingestellt und/oder geregelt werden. Beispielsweise kann das erste Fluid ein Aerosol aus flüssigem Wasser und einem Trägergas enthalten. In anderen Ausführungsformen der Erfindung kann das erste Fluid gasförmiges Wasser enthalten. Das zweite Fluid kann beispielsweise Sauerstoff oder ein gasförmiger bzw. in Form eines Aerosols vorliegender Duftstoff sein. Die Massenströme, welche jeweils durch die Austrittsöffnungen austreten, können durch den Benutzer einstellbar sein. In anderen Ausführungsformen der Erfindung kann mittels einer Gasanalyse der Massenstrom durch die zumindest eine Öffnung auf einen vorgebbaren Sollwert geregelt werden.

In einigen Ausführungsformen der Erfindung kann die erste Öffnung und/oder die zweite Öffnung und/oder eine Zufuhrleitung eine Düse mit einer Querschnittsverengung aufweisen. In anderen Ausführungsformen der Erfindung kann die zumindest eine Öffnung mit einem Ventil verschlossen sein. Diese Maßnahmen reduzieren oder vermindern den Rückstrom von Luft bzw. Gasen in den Arm bzw. die mit dem Arm verbundenen Zufuhrleitungen. Hierdurch wird eine Kontamination des Systems durch den Benutzer verhindert oder reduziert. In einigen Ausführungsformen der Erfindung wird die Strömungsgeschwindigkeit hinter der Düse wieder reduziert, um ein Anblasen des Gesichtes des Benutzers zu vermeiden, welches meist als unangenehm empfunden wird.

In einigen Ausführungsformen kann durch die zweite Öffnung und eine damit verbundene Rohrleitung ein Fluid vom Benutzer abgeleitet werden. Beispielsweise kann dieses Fluid die Ausatemluft des Benutzers sein. Auf diese Weise kann das dem Benutzer zugeführte erste Fluid eine wechselnde Zusammensetzung aufweisen, welche in Abhängigkeit des Analyseergebnisses des zweiten Fluides variieren.

In einigen Ausführungsformen kann die Vorrichtung weiterhin eine Einrichtung zur Erkennung des Atemrhythmus des Benutzers enthalten oder mit einer solchen verbunden sein. Auf diese Weise kann das zumindest eine Fluid dem Benutzer zyklisch zugeführt werden. Da die Zufuhr des Fluides nur in der Einatemphase erfolgt und nicht kontinuierlich auch während des Ausatmens, kann der Verbrauch der Fluide weiter reduziert werden. Insbesondere kann auf diese Weise die mit dem Flugzeug zu transportierende Wassermenge zur Befeuchtung der Atemluft weiter reduziert werden. In einigen Ausführungsformen der Erfindung kann das Erkennen des Atemrhythmus durch Detektion des Druckabfalls vor der Austrittsöffnung erfolgen, welcher sich durch das Einatmen des Benutzers ergibt. In einigen Ausführungsformen der Erfindung kann das Erkennen des Atemrhythmus durch Detektion des Ausatemstoßes erfolgen. Eine solche Erkennung ist zuverlässiger als die Erkennung des Einatmens anhand eines Druckabfalles. Im letzteren Fall wird das zumindest eine Fluid mit einer einstellbaren Zeitverzögerung nach Erkennung des Atemstoßes ausgestoßen.

In einigen Ausführungsformen der Erfindung kann die Vorrichtung weiterhin ein Mikrofon enthalten. In einigen Ausführungsformen kann das Mikrofon im Austrittsteil angeordnet sein. Auf diese Weise kann die vorgeschlagene Vorrichtung auch zur Kommunikation zwischen mehreren Benutzern dienen oder als Freisprecheinrichtung mit einem Mobiltelefon oder einem Funkgerät verbunden sein.

In einigen Ausführungsformen kann die Vorrichtung mit einer Einrichtung zur Abgabe eines Aerosols und/oder gasförmigen Wassers verbunden sein, welche in einem Fahr- oder Flugzeug fest eingebaut ist. In anderen Ausführungsformen der Erfindung kann die Einrichtung zur Abgabe eines Aerosols und/oder gasförmigen Wassers auch tragbar ausgeführt sein, so dass der Benutzer einen für eine bestimmte Zeitspanne ausreichenden Vorrat mit sich führen kann. Auf diese Weise wird der Benutzer von Infrastruktureinrichtungen eines Fahrzeuges, eines Flugzeuges oder eines Gebäudes unabhängig.

Nachfolgend soll die Erfindung anhand zweier Figuren ohne Beschränkung des allgemeinen Erfindungsgedankens näher erläutert werden. Dabei zeigt:
- Figur 1: einen Benutzer, welcher die erfindungsgemäß vorgeschlagene Vorrichtung trägt.
- Figur 2: zeigt eine genauere Darstellung des Austrittsteils der vorgeschlagenen Vorrichtung.

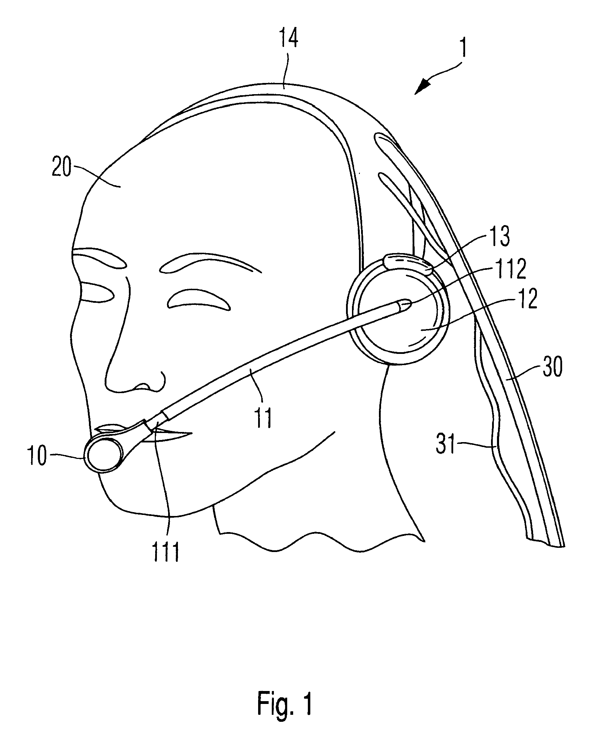

Figur 1 zeigt einen Benutzer 20, welcher die vorgeschlagene Vorrichtung 1 trägt. Die Vorrichtung 1 umfasst eine Kopfhalterung 14, welche sich von einer Ohrregion des Benutzers 20 cranial zur anderen Ohrregion des Benutzers 20 erstreckt. In anderen Ausführungsformen der Erfindung kann die Kopfhalterung 14 auch in anderer Weise von einer Ohrregion des Benutzers zur anderen Ohrregion des Benutzers verlaufen, beispielsweise dorsal des Kopfes.

An jedem Ende der Kopfhalterung 14 kann ein Schallwandler 12 angeordnet sein. Mit dem Schallwandler 12 kann ein Audiosignal wiedergegeben werden, beispielsweise wenn der Benutzer 20 mittels eines Funkgerätes oder eines Mobiltelefons kommunizieren möchte. In anderen Ausführungsformen der Erfindung können die Audiodaten auch ein Unterhaltungsprogramm übermitteln, beispielsweise eine Musikdarbietung. In einer weiteren Ausführungsform der Erfindung kann das über den zumindest einen Schallwandler 12 wiedergegebene Audiosignal amplitudengleich und phasenverschoben zu einem Umgebungsgeräusch sein, so dass der Benutzer 20 die Umgebungsgeräusche mit geringerer Lautstärke wahrnimmt. Audiosignale, welche mittels des zumindest einen Schallwandlers 12 wiedergegeben werden sollen, können über das Anschlusskabel 31 von einer Signalquelle zur Vorrichtung 1 übertragen werden.

Zwischen dem zumindest einen Schallwandler und dem Ohr des Benutzers kann ein Polsterelement 13 angeordnet sein, um den Tragekomfort des Schallwandlers 12 zu verbessern.

An der Kopfhalterung 14 befindet sich weiterhin ein Arm 11. Der Arm 11 weist ein erstes Ende 111 und ein zweites Ende 112 auf. Das zweite Ende 112 ist an der Kopfhalterung 14 befestigt. In einigen Ausführungsformen der Erfindung kann die Befestigung des zweiten Endes 12 an der Kopfhalterung 14 zumindest in einer Achse beweglich sein, so dass der Arm 11 drehbar bzw. schwenkbar gelagert ist. Der Arm 11 kann aus einem flexiblen Material hergestellt sein, so dass der Krümmungsradius durch den Benutzer mittels Verbiegen einstellbar ist. In einigen Ausführungsformen der Erfindung kann der Arm 11 aus zwei ineinander gesteckten Teilstücken bestehen, welche entlang ihrer Längsachse verschiebbar sind. Auf diese Weise kann auch die Länge des Armes 11 durch den Benutzer in einfacher Weise eingestellt werden. Der Arm 11 ist zumindest teilweise hohl ausgebildet, so dass ein Fluid entlang des Armes 11 vom zweiten Ende 112 zum ersten Ende 111 strömen kann. In anderen Ausführungsformen kann zumindest eine hohle Leitung entlang eines Armes 11 verlaufen.

Am ersten Ende 111 des Armes 11 befindet sich ein Austrittsteil 10. Das Austrittsteil 10 kann mit dem ersten Ende 111 des Armes 11 lösbar verbunden sein, so dass das Austrittsteil 10 in einfacher Weise austauschbar ist oder zur Reinigung abgenommen werden kann. Die Verbindung zwischen dem Austrittsteil 10 und dem Arm 11 kann beispielsweise mittels eines Gewindes, eines Bajonettverschlusses oder eines Schiebesitzes erfolgen.

Zumindest ein erstes Fluid, welches beispielsweise Wasser, Sauerstoff, ein Trägergas oder einen Duftstoff enthält, wird mittels der Zufuhrleitung 30 der Vorrichtung 1 zugeführt. Die Zufuhrleitung 30 ist an ihrem anderen Ende in einigen Ausführungsformen der Erfindung mit einem nicht dargestellten Gasreservoir verbunden. In anderen Ausführungsformen der Erfindung kann eine Einrichtung vorgesehen sein, welche ein flüssiges Medium in den gasförmigen Zustand oder in ein Aerosol überführt. Das flüssige Medium kann beispielsweise Wasser sein. Die Überführung in einen gasförmigen Zustand bzw. in ein Aerosol kann in einigen Ausführungsformen der Erfindung durch Ultraschallvernebelung, mittels Verdampfung, durch Expansion durch eine Düse oder durch weitere Verfahren erfolgen. Das gasförmige Wasser kann dann durch die Leitung 30 zur Vorrichtung 1 geleitet werden. Hierzu kann ein Trägergas verwendet werden, beispielsweise Umgebungsluft.

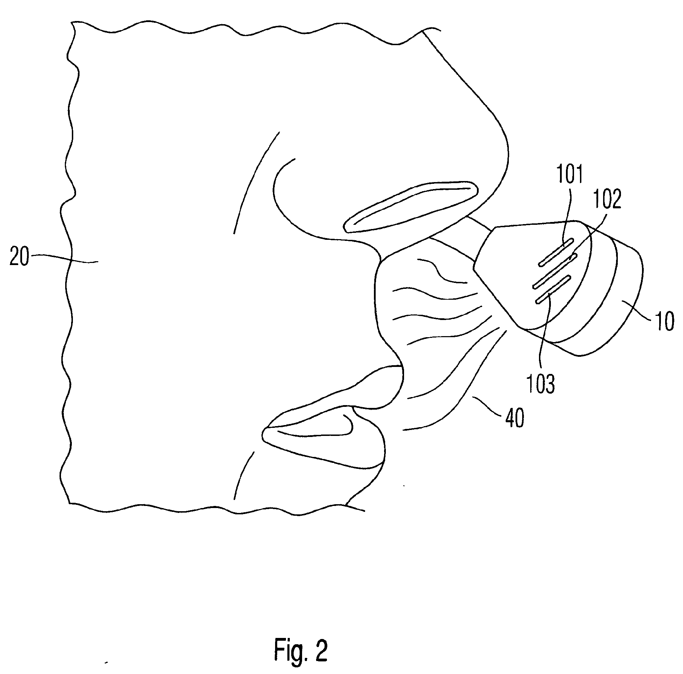

Figur 2 zeigt eine genauere Ansicht des Austrittsteiles 10. Im linken Bildteil der Figur 2 ist ein Ausschnitt aus dem Gesicht des Benutzers 20 dargestellt. Die Länge und/oder der Winkel und/oder die Krümmung des Armes 11 sind so eingestellt, dass das Austrittsteil 10 zwischen Mund und Nase des Benutzers 20 in einem Abstand von etwa 2 cm positioniert ist. Auf der dem Benutzer zugewandten Seite des Austrittsteiles 10 befindet sich eine erste Öffnung 101 und eine zweite Öffnung 102. Die erste Öffnung 101 findet Anschluss an ein entlang des Armes 11 verlaufendes Rohrsystem und die Zufuhrleitung 30. Auf diese Weise kann das über die Anschlussleitung 30 zugeführte erste Fluid aus der ersten Öffnung 101 austreten. Im dargestellten Ausführungsbeispiel wird als erstes Fluid Wasser zugeführt, um die Atemluft des Benutzers 20 zu befeuchten. Das erste Fluid bildet dann im Bereich vor den Atemöffnungen des Benutzers 20 einen Dampf bzw. ein Aerosol 40. Aufgrund der Befestigung der Vorrichtung 1 am Kopf des Benutzers 20 folgt der Bereich, in welchem sich das Aerosol 40 ausbildet, den Bewegungen des Benutzers. Dadurch wird die Bewegungsfreiheit des Benutzers erhöht.

Um den Verbrauch an Fluid zu reduzieren, wird das erste Fluid 40 aus der ersten Öffnung 101 nur dann ausgestoßen, wenn der Benutzer 20 einatmet. Zur Erkennung des Atemzyklus befindet sich hinter der Öffnung 102 ein Sensor, welcher den Atemstoß des Benutzers 20 beim Ausatmen detektiert. Durch eine Logikschaltung wird veranlasst, dass das erste Fluid aus der ersten Öffnung 101 stets im Zeitraum zwischen zwei erkannten Atemstößen ausgestoßen wird. Dies bedeutet, dass der Ausstoß des Fluids aus der ersten Öffnung 101 mit einer vorgebbaren Verzögerung nach Erkennung des Atemstoßes mittels des Sensors hinter der Öffnung 102 erfolgt. Als Sensor kann in einigen Ausführungsformen ein Drucksensor und/oder ein Luftgeschwindigkeitssensor und/oder ein Temperatursensor und/oder CO2-Sensor und/oder ein Luftqualitätssensor eingesetzt werden. Sofern das Austrittsteil 10 ein Mikrophon enthält, um die Stimme des Benutzers aufzuzeichnen, kann dieses zusätzlich zur Erkennung des Atemrhythmus eingesetzt werden.

Weiterhin befindet sich im Austrittsteil 10 eine dritte Öffnung 103. Hinter der dritten Öffnung 103 kann in einigen Ausführungsformen der Erfindung ein Mikrofon angeordnet sein, welches die Stimme des Benutzers 20 aufzeichnen kann. Auf diese Weise kann der Benutzer 20 mit anderen Benutzern einer Vorrichtung 1 über ein kabelgebundenes oder funkgekoppeltes Kommunikationssystem kommunizieren. In anderen Ausführungsformen der Erfindung kann das Mikrofon dazu dienen, die Stimme des Benutzers über ein Mobiltelefon oder ein Funkgerät zu übertragen.

Die vorstehende Beschreibung ist so zu verstehen, dass ein genanntes Merkmal in zumindest einer Ausführungsform der Erfindung vorhanden ist. Dies schließt die Anwesenheit weiterer Merkmale nicht aus. Sofern die Ansprüche "erste" und "zweite" Merkmale definieren, so dient diese Bezeichnung der Unterscheidung zweier gleichartiger Merkmale, ohne eine Rangfolge festzulegen.

Selbstverständlich können die dargestellten Ausführungsbeispiele verändert werden, um auf diese Weise weitere, unterschiedliche Ausführungsformen der Erfindung zu erhalten. Die vorstehende Beschreibung ist daher nicht als beschränkend, sondern als erläuternd anzusehen.

## Patentansprüche

1. Vorrichtung (1) zum Befeuchten der Atemluft eines Benutzers (20), enthaltend
• eine Kopfhalterung (14), welche am Kopf des Benutzers (20) angeordnet werden kann, und welche sich von einer Ohrregion des Benutzers (20) zur anderen Ohrregion des Benutzers (20) erstreckt;
∘ einen Arm (11), aufweisend ein erstes Ende (111) und ein zweites Ende (112), wobei das zweite Ende (112) an der Kopfhalterung (14) befestigt ist, und sich das erste Ende (111) beim Betrieb der Vorrichtung in Richtung des Nasen- und/oder Mundbereiches des Benutzers (20) erstreckt; und
∘ ein Austrittsteil (10), welches mit dem ersten Ende (111) des Armes (11) verbindbar ist, und welches zumindest eine erste Öffnung (101) enthält, durch welche ein erstes Fluid zu dem Benutzer geleitet werden kann,
**dadurch gekennzeichnet, dass**
∘ weiterhin enthaltend eine Einrichtung zur Erkennung des Atemrhythmus des Benutzers (20).

2. Vorrichtung nach Anspruch 1, **dadurch gekennzeichnet, dass** die Vorrichtung zwei Schallwandler (12) enthält, welche jeweils an gegenüberliegenden Enden der Kopfhalterung (14) befestigt sind, so dass diese bei Benutzung der Vorrichtung an jeweils einem Ohr des Benutzers zu liegen kommen.

3. Vorrichtung nach einem der Ansprüche 1 oder 2, **dadurch gekennzeichnet, dass** der Arm (11) schwenkbar an der Kopfhalterung (14) befestigt ist und/oder seine Länge durch den Benutzer (20) veränderbar ist.

4. Vorrichtung nach einem der Ansprüche 1 bis 3, **dadurch gekennzeichnet, dass** das Austrittsteil (10) zumindest eine zweite Öffnung (102) enthält, durch welche ein zweites Fluid zu dem Benutzer geleitet werden kann.

5. Vorrichtung nach Anspruch 4, **dadurch gekennzeichnet, dass** die Einrichtung zur Erkennung des Atemrhythmus einen Drucksensor und/oder einen Luftgeschwindigkeitssensor und/oder einen Temperatursensor und/oder ein einen CO₂-Sensor und/oder einen Luftqualitätssensor enthält.

6. Vorrichtung nach einem der Ansprüche 1 bis 5, **dadurch gekennzeichnet, dass** an der ersten Öffnung (101) und/oder der zweiten Öffnung (102) und/oder in der Zufuhrleitung des Fluids eine Düse angeordnet ist, welche eine temporäre Erhöhung der Strömungsgeschwindigkeit des Fluids bewirkt.

7. Vorrichtung nach einem der Ansprüche 1 bis 6, **dadurch gekennzeichnet, dass** das Austrittsteil (10) weiterhin ein Mikrofon (103) enthält.

8. Vorrichtung nach einem der Ansprüche 1 bis 7, weiterhin enthaltend eine Einrichtung zur Abgabe eines Aerosols (40) und/oder gasförmigen Wassers.

9. Vorrichtung nach einem der Ansprüche 1 bis 8, weiterhin enthaltend zumindest einen Schallwandler (12), mit welchem zumindest ein Audiosignal wiedergegeben werden kann.

10. Fahr- oder Flugzeug mit einer Vorrichtung nach einem der Ansprüche 1 bis 9.

## Claims

1. Device (1) for humidifying the respiratory air of a user (20), containing
∘ a head support (14), which can be arranged at the head of the user (20) and which extends from one ear region of the user (20) to the other ear region of the user (20) ;
∘ an arm (11) having a first end (111) and a second end (112), the second end (112) being mounted on the head support (14) and the first end (111) extending in the direction of the nose and/or mouth region of the user (20) while the device is operated; and
∘ an outlet member (10), which can be connected to the first end (111) of the arm (11) and which contains at least one first opening (101), through which a first fluid can be guided to the user,
**characterized by**
∘ further containing an apparatus for detecting the breathing rhythm of the user (20).

2. Device according to claim 1, **characterized in that** the device contains two sound transducers (12), which are mounted on respectively opposite ends of the head support (14), such that each ear of the user is provided with a transducer when the device is used.

3. Device according to any of claims 1 or 2, **characterized in that** the arm (11) is pivotably mounted on the head support (14) and/or the length thereof can be modified by the user (20).

4. Device according to any of claims 1 to 3, **characterized in that** the outlet member (10) contains at least one second opening (102), through which a second fluid can be guided to the user.

5. Device according to claim 4, **characterized in that** the apparatus for detecting the breathing rhythm contains a pressure sensor and/or an air velocity sensor and/or a temperature sensor and/or a CO₂ sensor and/or an air quality sensor.

6. Device according to any of claims 1 to 5, **characterized in that** a nozzle is arranged at the first opening (101) and/or the second opening (102) and/or in the supply line of the fluid and effects a temporary increase in the flow rate of the fluid.

7. Device according to any of claims 1 to 6, **characterized in that** the outlet member (10) further contains a microphone (103).

8. Device according to any of claims 1 to 7, further containing an apparatus for dispensing an aerosol (40) and/or gaseous water.

9. Device according to any of claims 1 to 8, further containing at least one sound transducer (12), by means of which at least one audio signal can be reproduced.

10. Vehicle or aircraft comprising a device according to any of claims 1 to 9.

## Revendications

1. Dispositif (1) pour l'humidification de l'air respiratoire d'un utilisateur (20), comprenant
- un serre-tête (14) susceptible d'être agencé sur la tête de l'utilisateur (20) et qui s'étend depuis une zone d'une oreille de l'utilisateur (20) jusqu'à la zone de l'autre oreille de l'utilisateur (20) ;
- un bras (11) pourvu d'une première extrémité (111) et d'une seconde extrémité (112), la seconde extrémité (112) étant fixée sur le serre-tête (14) et la première extrémité (111) s'étendant en direction de la zone du nez et/ou de la bouche de l'utilisateur (20), le dispositif étant en fonctionnement ; et
- une partie de sortie (10) qui est susceptible d'être reliée à la première extrémité (111) du bras (11) et qui présente au moins une première ouverture (101) à travers laquelle un premier fluide peut être amené à l'utilisateur,
**caractérisé en ce que**
- il comprend en outre un moyen pour détecter le rythme respiratoire de l'utilisateur (20).

2. Dispositif selon la revendication 1, **caractérisé en ce que** le dispositif comprend deux transducteurs acoustiques (12) qui sont fixés respectivement à des extrémités opposées du serre-tête (14) de manière à venir se poser chacun sur une oreille de l'utilisateur lorsqu'il utilise le dispositif.

3. Dispositif selon l'une des revendications 1 ou 2, **caractérisé en ce que** le bras (11) est fixé avec faculté de pivotement sur le serre-tête (14) et/ou sa longueur est variable par l'utilisateur (20).

4. Dispositif selon l'une des revendications 1 à 3, **caractérisé en ce que** la partie de sortie (10) comprend au moins une seconde ouverture (102) à travers laquelle un second fluide peut être amené à l'utilisateur.

5. Dispositif selon la revendication 4, **caractérisé en ce que** le moyen de détection du rythme respiratoire comprend un capteur de pression et/ou un capteur de vitesse d'air et/ou un capteur de température et/ou un capteur de CO₂ et/ou un capteur de qualité d'air.

6. Dispositif selon l'une des revendications 1 à 5, **caractérisé en ce qu'**une buse est agencée sur la première ouverture (101) et/ou sur la seconde ouverture (102) et/ou dans la conduite d'alimentation du fluide, qui provoque une augmentation temporaire de la vitesse d'écoulement du fluide.

7. Dispositif selon l'une des revendications 1 à 6, **caractérisé en ce que** la partie de sortie (10) comprend en outre un microphone (103).

8. Dispositif selon l'une des revendications 1 à 7, comprenant en outre un moyen pour distribuer un aérosol (40) et/ou de l'eau sous forme gazeuse.

9. Dispositif selon l'une des revendications 1 à 8, comprenant en outre un transducteur acoustique (12) qui permet de reproduire un signal sonore.

10. Véhicule ou avion pourvu d'un dispositif selon l'une des revendications 1 à 9.
